# EUROPEAN PATENT APPLICATION

(11) **EP 2 604 596 A1**
(43) Date of publication of application: **19.06.2013**
(21) Application number: 11193930.2
(22) Date of filing: 16.12.2011
(51) Int. Cl.: C07D 401/04, A61K 31/506

(54) **Polymorphs of imatinib**

(71) Applicant: Deva Holding Anonim Sirketi, Kucukcekmece/Istanbul (TR)
(72) Inventor: Haas, Philipp Daniel, 34303 ISTANBUL (TR); Koc, Fikret, 34303 ISTANBUL (TR); Karliga, Bekir, 34303 ISTANBUL (TR); Bellur Atici, Esen, 34303 ISTANBUL (TR); Sivasligil, Ramazan, 34303 ISTANBUL (TR)

(57) **Abstract**

This invention relates to novel, stable, highly soluble M, Y, B, P polymorphs of imatinib mesylate and process for preparation thereof and pharmaceutical compositions comprising imatinib mesylate.

## Description

### Technical Field

This invention relates to novel, stable, highly soluble, polymorphs of M, Y, B, P of imatinib mesylate and process for preparation thereof and pharmaceutical compositions comprising imatinib mesylate.

### Background Art

Imatinib mesylate, is chemically 4-[(4-Methyl-1-piperazinyl) methyl]-N-[4-methyl-3-[[4-(3-pyridinyl)-2-pyrimidinyl] amino] phenyl]-benzamide mesylate. It is represented by the following formula;

Imatinib is the first member of a new class of agents that act specially by inhibiting a certain enzyme that is characteristic of a particular cancer cell.

Imatinib is a protein-tyrosine inhibitor used to treat certain types of cancer; it is especially used to treat chronic myelogenous leukemia (CML) and gastrointestinal stromal tumors (GISTs).

WO 95/09852 A (CIBA-GEIGY A.G.) 13.04.1995 discloses firstly imatinib.

Polymorphism is defined as the ability of a substance to exist as two or more crystalline phases that have different arrangements or conformations of the molecules in the crystal lattice.

WO 99/03854 A (NOVARTIS-ERFINDUNGEN VERWALTUNGSGESELLSCHAFT MBH) 28.01.1998 discloses two crystalline α crystalline and β crystalline forms of imatinib mesylate.

WO 2004/106326 A (HETERO DRUGS LIMITED) 09.12.2004 discloses a crystalline imatinib mesylate form H1.

### Summary of invention

This invention relates to new crystalline forms of imatinib mesylate and processes for preparation thereof and pharmaceutical compositions of imatinib mesylate.

### Technical Problem

Recently, in solid state pharmaceutical science, development and characterization of new crystal forms of active pharmaceutical ingredients is an ongoing challenge and it is a routine practice to investigate in development for new candidates of polymorphism for active pharmaceutical ingredients.

Since the discovery of new crystalline forms of an active pharmaceutical ingredient provides an opportunity to improve the performance characteristics of pharmaceutical finished product, the development of new polymorphic forms is always encouraged.

The most important physical property is the flowability of active pharmaceutical ingredient which affects the ease with which the material is handled during manufacturing of a pharmaceutical product. Another important solid state physical property of a pharmaceutical compound is its rate of dissolution in aqueous fluid.

Moreover, the solid state form of an active pharmaceutical ingredient may affect its behaviour on compaction and its storage stability as finished product.

Furthermore, solid state study of an active pharmaceutical ingredient aims to widen the variety of crystalline forms that a formulation scientist has available for designing a pharmaceutical dosage with desired characteristic.

Imatinib is the first cancer agent proven effective for metastatic GIST, its phenomenal success is frequently mentioned in the treatment of this disease since its market introduction.

Consequently, there is a constant need for easy, simple, economic, reliable processes for the preparation of new polymorphic forms of imatinib mesylate which may be used for commercial manufacturing of capsule or tablets.

### Solution to Problem

The performance characteristics of a pharmaceutical product can be improved by means of discovery of new polymorphic forms.

Nevermore, simplicity of the preparation process, improvement of productivity and increase of process yield are three main reasons to motivate the inventor to search for new polymorphic forms.

Besides, controlling the conditions under which solid forms is extremely important in developing polymorphic forms. Mode of isolation and solvent medium rank as dependent variable in the control of conditions.

In the present invention, it is aimed to develop a simple, reproducible process method with better yield in the preparation of imatinib mesylate.

The preparation of new polymorphic forms of imatinib mesylate is effectuated with the reaction of equimolar amount of imatinib base and methanesulfonic acid in a ketone, alcohol, ether and cyclic ether.

Inventors have surprisingly found out new, stable crystalline forms of imatinib mesylate. And the newly obtained crystalline forms have features to be stable at room and accelerated stress conditions over time.

In addition, due to their low hygroscopicity, polymorphs of mixture, Y, B, α+ β of imatinib mesylate have shown superior flow properties which make these crystalline forms highly suitable in formulation of imatinib mesylate.

The advantageous technical effect of the polymorph of mixture, Y, B, α+ β are proven according to comparative solubility, stability tests.

### Brief description of drawings

Fig.1 provides the X-ray diffractogram of crystalline M form of imatinib mesylate prepared according to the process of the present invention.

Fig.2 provides the infrared spectrum (IR) of crystalline M form of imatinib mesylate prepared according to the process of the present invention.

Fig.3 provides the differential scanning thermogram (DSC) of crystalline M form of imatinib mesylate prepared according to the process of the present invention.

Fig.4 provides the X-ray diffractogram of crystalline Y form of imatinib mesylate prepared according to the process of the present invention.

Fig.5 provides the infrared spectrum (IR) of crystalline Y form of imatinib mesylate prepared according to the process of the present invention.

Fig.6 provides the differential scanning thermogram (DSC) of crystalline Y form of imatinib mesylate prepared according to the process of the present invention.

Fig.7 provides the X-ray diffractogram of crystalline B form of imatinib mesylate prepared according to the process of the present invention.

Fig.8 provides the infrared spectrum (IR) of crystalline B form of imatinib mesylate prepared according to the process of the present invention.

Fig.9 provides the differential scanning thermogram (DSC) of crystalline B form of imatinib mesylate prepared according to the process of the present invention.

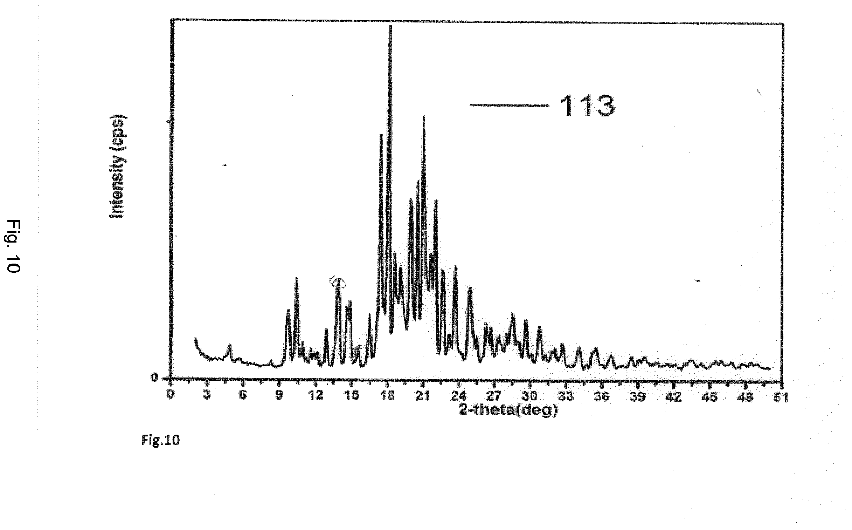

Fig.10 provides the X-ray diffractogram of crystalline P form of imatinib mesylate prepared according to the process of the present invention.

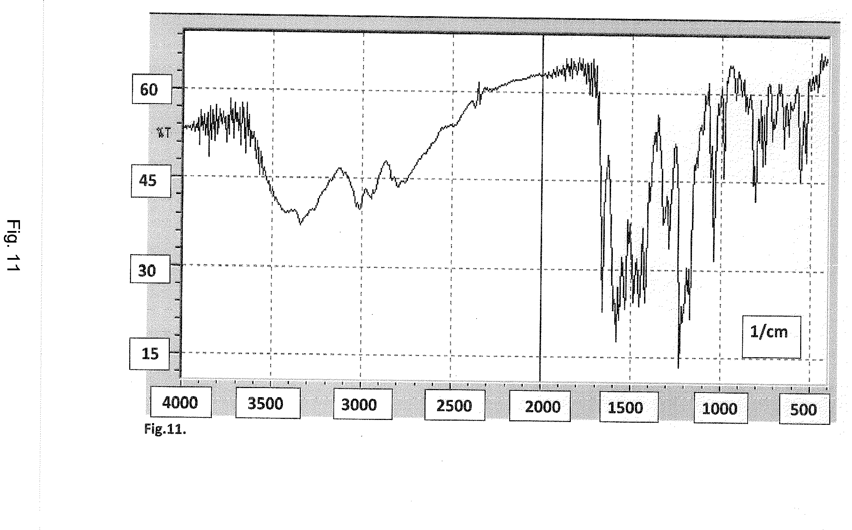

Fig.11 provides the infrared spectrum (IR) of crystalline P form of imatinib mesylate prepared according to the process of the present invention.

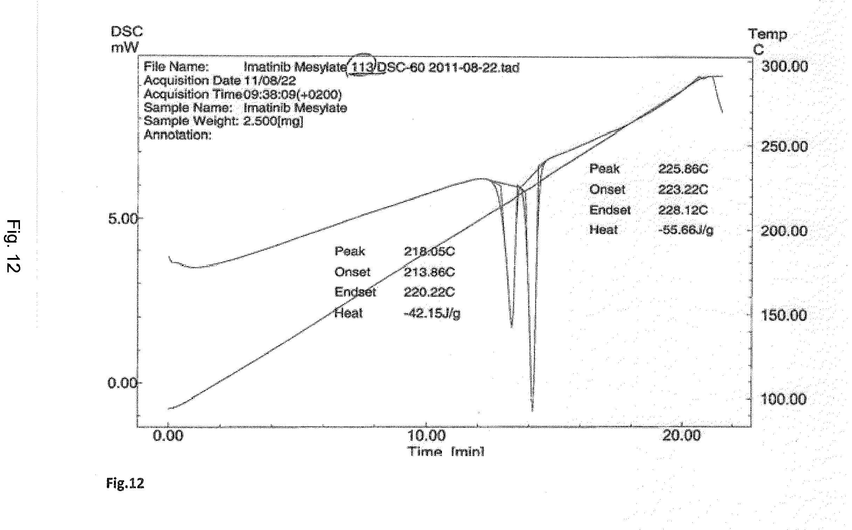

Fig.12 provides the differential scanning thermogram (DSC) of crystalline P form of imatinib mesylate prepared according to the process of the present invention.

### Description of embodiments

The main objective of the present invention is to provide novel crystalline forms of imatinib mesylate. Crystalline M form, crystalline Y form, crystalline B form, crystalline P form which are prepared by using rational design to control adequately polymorphism, are presented.

Presented each crystalline forms give rise to distinct spectroscopic properties that may be detectable by powder X-ray crystallography and infrared spectrometry. The new polymorphic forms of imatinib mesylate are evaluated by differential scanning calorimetry diagrams as well.

X-ray diffraction data were acquired using a Rigaku smart X-ray diffractometer model Smartlab. System description: K_{α1}=1.54178Å, voltage 40 kV, current 30 mA, incident slit 1/2°,receiving slit #1=1/2°,receiving slit #2=0.2mm, receiving paralel slit=sollerslit 5.0, incident paralel slit=sollerslit 5.0°, with a Graphite monochromator. Measurements of 2θ values are typically accurate to within ±0.002 degrees. Experiment parameters: pattern measured between 2θ=3°and 2θ=50° with 0.1 ° increments; count time was 2 second per increment.

Infrared spectroscopy is one of the most commonly used spectroscopic techniques where the chemical functional groups of the sample are determined. Structural elucidation and compound identification are obtained with infrared spectroscopy during the study of polymorphic forms.

The infrared (IR) spectrum has been recorded on a Shimadzu Fourrier-transform infra-red spectrometer model IR Prestige 21 within the range of 400 and 4000 cm⁻¹ and resolution of 2 cm⁻¹. All samples were run as KBr disks.

The infrared spectrum of Form as a KBr tablet has the characteristic absorptions at the following wavelenghts in cm-1.

Thermal behaviour is measured in the laboratory by differential scanning calorimetry (DSC) and may be used to distinguish some polymorphic forms from others.

Differential scanning calorimetry (DSC) measurements were run on Shimadzu model DSC 60. Samples were analyzed inside crimped 40 µl aluminium pans. The temperature range is 100-300°C. Heating rate for all samples was 10°C/min.

Another objective of the invention is to provide processes for the preparation of novel crystalline polymorphic forms of imatinib mesylate.

In the first aspect of this invention, the preparation of polymorph M of imatinib mesylate in the reaction of equimolar amount of imatinib base and methanesulfonic acid in a ketone is presented.
Preparation process of M polymorph according to the invention is characterized by:

(a) Suspending or dissolving imatinib base in a ketone.
(b) heating the mixture for reflux temperature,

(c) adding methanesulfonic acid, at a controlled rate, diluted with solvent, to the reaction mixture,

(d) cooling the mixture to ambient temperature and isolating the mixture crystal form;

(e) isolating the crystalline product from the reaction mixture.

(f) drying the isolated product under vacuum at temperature from 50 to 65°C.

The quantity of methanesulfonic acid per mole of imatinib free base is not critical but preferably at least one mole of methanesulfonic acid per mole of imatinib free base is used to obtain maximum yield of imatinib mesylate.

Examples of ketones are acetone, propanone and methyl isobuthyl ketone. Preferable ketone is acetone.

As used herein, the term reflux temperature refers to a temperature from about 50°C to about 70°C

In step (b), the mixture is preferably heated to at least about 55 ° C, more preferably at least about 60°C, most preferably at least about 70° C.

Preferably the cooling to ambient temperature in step (d) involves cooling to 20-25°C.

The isolation of the crystalline product from the reaction mixture in step (e) may be achieved by filtration, preferably followed be drying under reduced pressure. Immediately after filtration, the filtrate is preferably dissolved or suspended in a solvent, such as acetone. The solvent may be the same as the solvent used in step (a).

In this aspect of the invention, provided herein is the M form of imatinib mesylate characterized by data selected from the group consisting of:

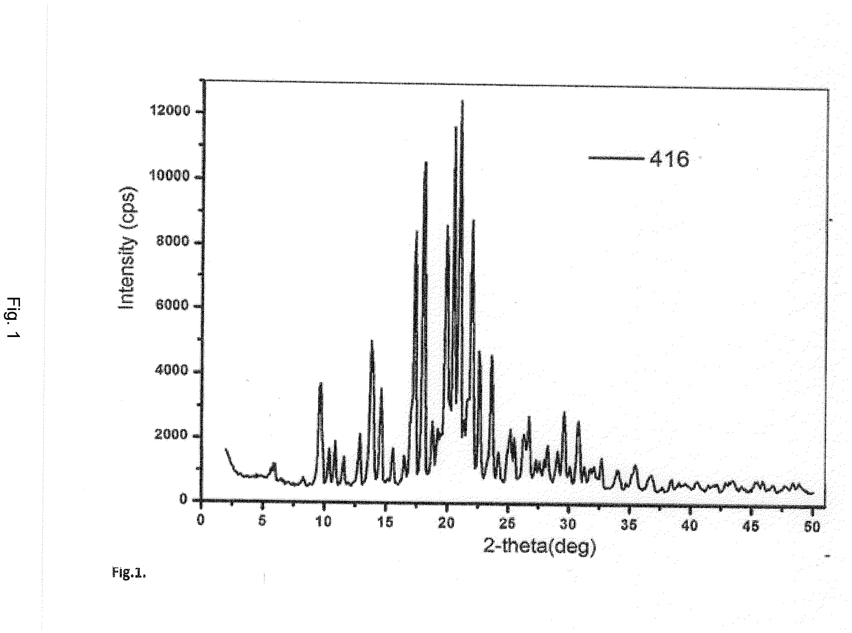

i) a X-ray powder diffraction pattern shown in Fig. 1.

ii) the powder X-ray diffraction pattern having peaks at about 5.97, 9.64,10.42,10.94,11.59,12.93,13,916,14.63,15.567,17.39,18.051,18.82,19. 90, 20.51, 21.04, 21.8, 21.97, 22.63, 23.66, 24.2, 26.23, 26.8, 28.3, 29.10, 29.61, 30.1, 30.76, 31.79, 32.76, 34.02, 35.52, 36.80, 40.5, 42.2,43.5 as depicted in Fig.1.

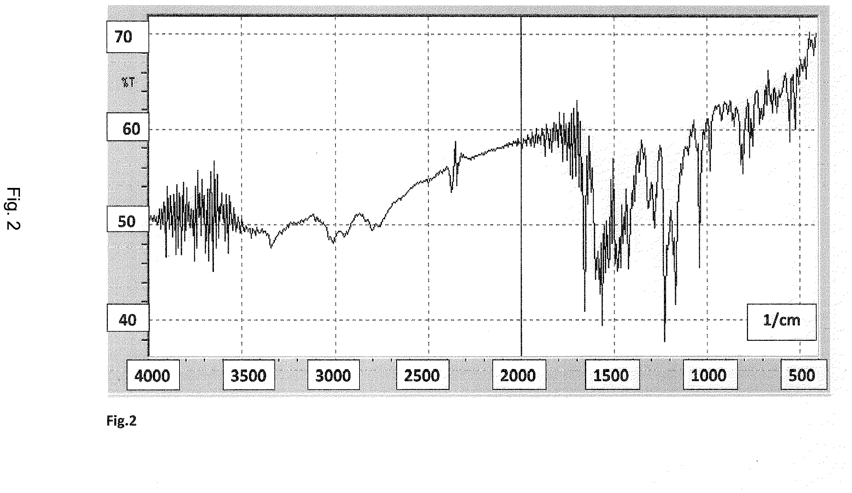

iii) the IR spectrum is substantially in accordance with Fig. 2,

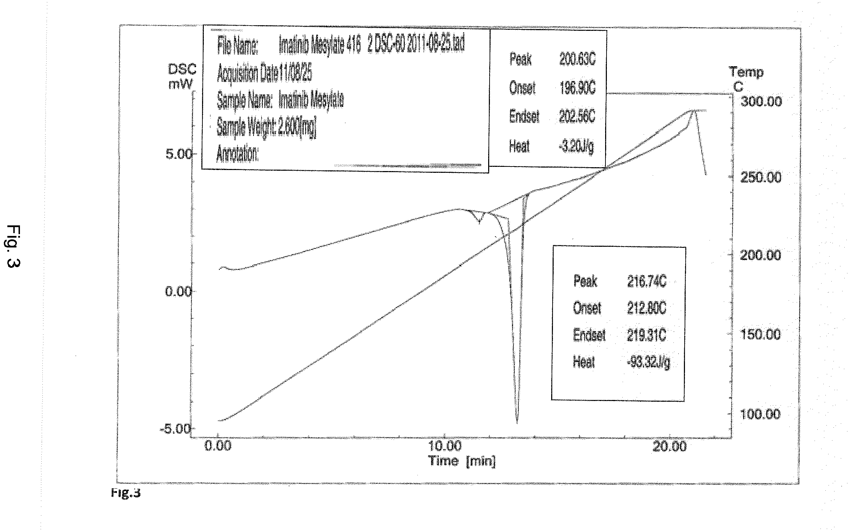

iv)a DSC thermogram substantially in accordance with Fig. 3,

v) the DSC thermogram shown in Fig.3 points with melting points: 216.74 and 200.63°C for polymorph mixture of imatinib mesylate.

In the second aspect of this invention is to provide polymorph Y of imatinib mesylate the preparation of polymorph Y of imatinib mesylate in the reaction of equimolar amount of imatinib base and methanesulfonic acid in an alcohol.
Preparation process of polymorph Y according to the invention is characterized by:

(a) suspending imatinib base in a alcohol and stirring the solution at temperature from -10 to 10°C,
(b) adding methanesulfonic acid, at a controlled rate, diluted with solvent, to the reaction mixture,
(c) stirring the reaction mixture at temperature from -10°C to 10°C,
(d) isolating the crystalline product from the reaction mixture,
(f) drying the isolated product under vacuum at temperature from 50 to 65°C.

The quantity of methanesulfonic acid per mole of imatinib free base is not critical but preferably at least one mole of methanesulfonic acid per mole of imatinib free base is used to obtain maximum yield of imatinib mesylate.

Examples of alcohols are ethanol, propanol and 2-propanol. Preferable alcohol is propanol.

The isolation of crystalline product from the reaction mixture, in step (d) may be achieved by filtration, preferably followed be drying under reduced pressure. Immediately after filtration, the filtrate is preferably dissolved or suspended in a solvent, such as a C₂ to C₄ alcohol. The solvent may be the same as the solvent used in step (a).

In this aspect of the invention, provided herein is the imatinib mesylate polymorph Y characterized by data selected from the group consisting of:

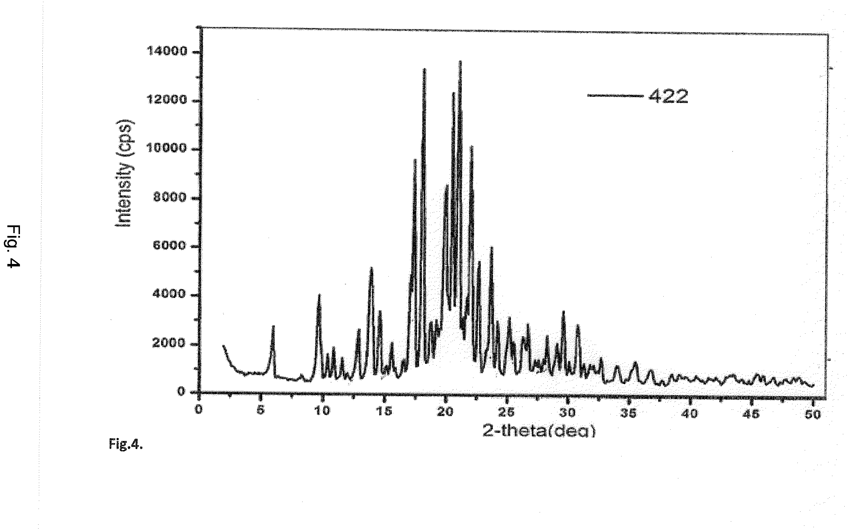

i) a X-ray powder diffraction pattern shown in Fig. 4.

ii)the powder X-ray diffraction pattern having peaks at about 6.017, 9.67,10.40, 10.95,11.6, 12.91, 13.950,14.640,17.09,17.4,18.09,19.97, 20.54, 21.03, 22.01, 23.66, 24.2, 26.23, 29.11, 29.61, 30.77, 31.74, 32.75, 34.05, 35.55, 36.81, 38.42, 39.02, 40.48, 43.46, 45.28, 48.9.as depicted in Fig.4.

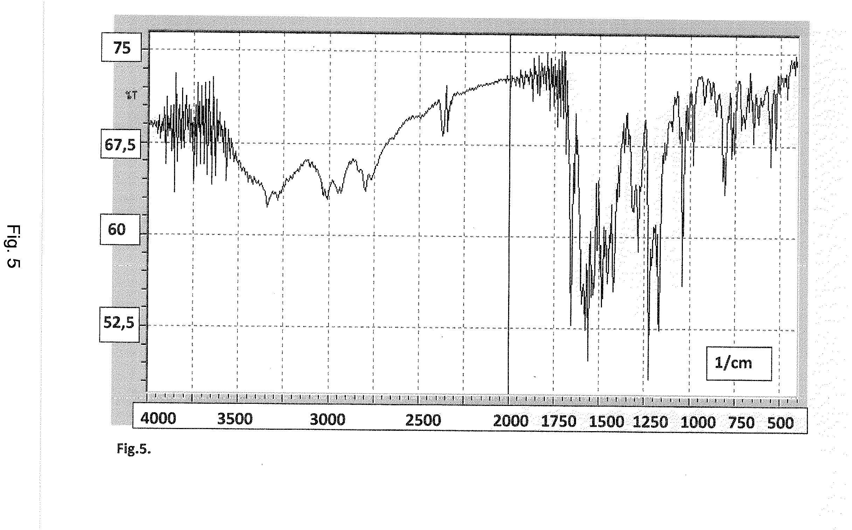

iii) the IR spectrum is substantially in accordance with Fig. 5,

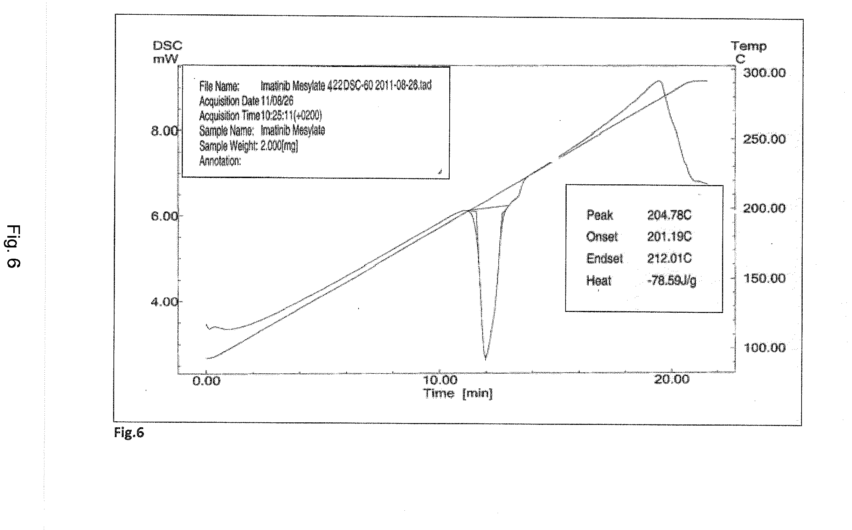

iv)a DSC thermogram substantially in accordance with Fig. 6,

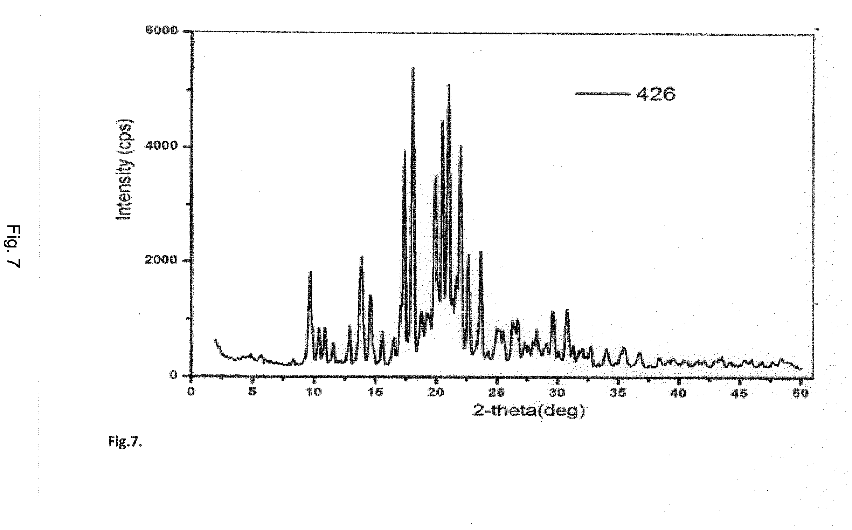

v) the DSC thermogram shown in Fig.7 points melting point: 204.78°C for polymorph Y of imatinib mesylate.

In a third aspect of the present invention is to provide polymorph B and the preparation of polymorph B of imatinib mesylate in the reaction of equimolar amount of imatinib base and methanesulfonic acid, in an ether is presented.
Preparation process of polymorph B according to the invention is characterized by:

(a) suspending or dissolving imatinib base in a ether and stirring the solution at room temperature,
(b) adding methanesulfonic acid, at a controlled rate, diluted with solvent, to the reaction mixture,
(c) stirring the reaction mixture at room temperature,

(d) heating the above mixture for a sufficient amount of time to induce the formation of new crystal form,

(e) cooling the mixture to ambient temperature and isolating the new crystal form;

(f) isolating the crystalline product from the reaction mixture,
(g) drying the isolated product under vacuum at temperature from 50 to 65°C.

In this aspect of the invention, provided herein is the polymorph B of imatinib mesylate characterized by data selected from the group consisting of:

i) a X-ray powder diffraction pattern shown in Fig. 7.

ii) a powder X-ray diffraction pattern having peaks at about 4.7, 9.67, 10.44, 10.95, 13.0, 13.92, 14.65, 17.39, 18.10, 19.96, 20.55, 21.04, 22.03, 22.6267, 23.68, 25.06, 26.27, 26.78, 28.3, 29.64, 30.75, 31.75, 32.78, 34.04, 35.56, 36.83.

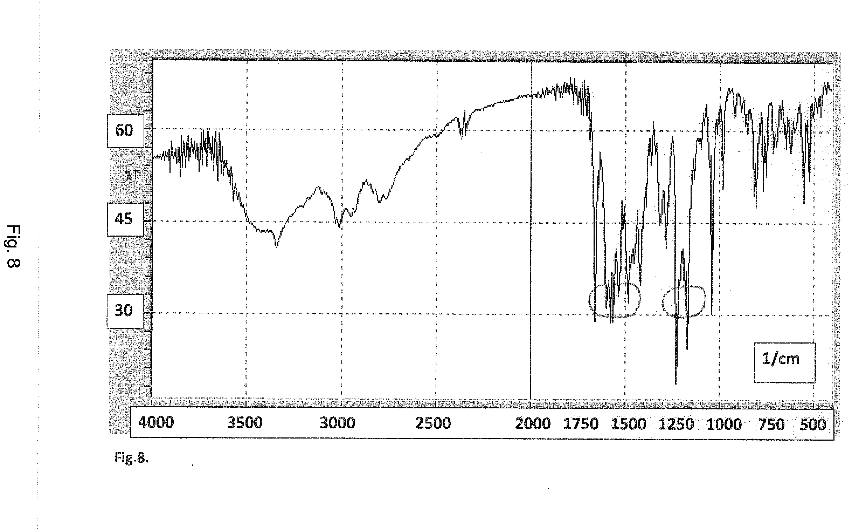

iii) the IR spectrum is substantially in accordance with Fig. 8,

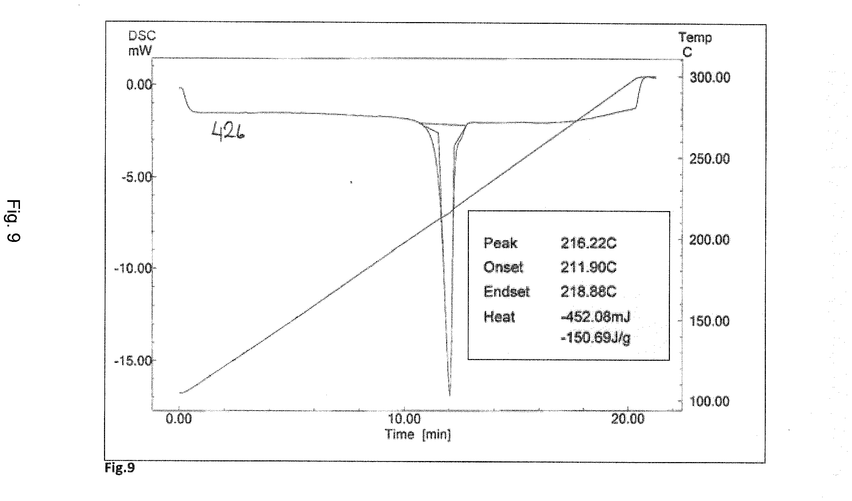

iv)a DSC thermogram substantially in accordance with Fig. 9,

v)DSC thermogram shown in Fig.7 points melting point: 216.22 for polymorph B of imatinib mesylate

In the fourth aspect of the present invention, the preparation of polymorph P of imatinib mesylate in the reaction of equimolar amount of imatinib base and methanesulfonic acid, in a cyclic ether is presented. Preparation process of polymorph P according to the invention is characterized by:

(a) Suspending or dissolving imatinib base in a ether and stirring the solution at room temperature,
(b) Stirring the reaction mixture at room temperature,

(c) Heating the above mixture for reflux temperature

(d) Adding alpha crystals of imatinib mesylate.

(e) Adding methanesulfonic acid, at a controlled rate, diluted with solvent, to the reaction mixture,

(f) Cooling the mixture to ambient temperature and isolating the mixture crystal form;

(g) Isolating the crystalline product from the reaction mixture,
(h) Drying the isolated product under vacuum at temperature from 50 to 65°C.

The quantity of methanesulfonic acid per mole of imatinib free base is not critical but preferably at least one mole of methanesulfonic acid per mole of imatinib free base is used to obtain maximum yield of imatinib mesylate.

Examples of cyclic ethers are THF and dioxane. Preferable cyclic ether is THF.

As used herein, the term room temperature refers to a temperature from about 20°C to about 30°C.

As used herein, the term reflux temperature refers to a temperature from about 50°C to about 70°C. Preferably the heating to reflux temperature in step (c) involves cooling to 55 - 60°C.

Preferably the cooling to ambient temperature in step (f) involves cooling to 25 - 30°C.

In step (c), the mixture is preferably heated to at least about 50° C, more preferably at least about 55°C, most preferably at least about 60° C.

The isolation of the crystalline product from the reaction mixture, in step (g) may be achieved by filtration, preferably followed be drying under reduced pressure. Immediately after filtration, the filtrate is preferably dissolved or suspended in a solvent, such as a THF. The solvent may be the same as the solvent used in step (a). Preferably the solvent is THF.

In this aspect of the invention, provided herein is the polymorph P of imatinib mesylate characterized by data selected from the group consisting of:

i) a X-ray powder diffraction pattern shown in Fig. 7.

ii)a powder X-ray diffraction pattern having peaks at about 4.92, 9.69, 10.45, 12.00, 12.95, 13.90, 14.70, 17.36, 18.06, 18.52, 19.08,19.93, 20.51, 20.97, 21.66, 22.0,24.85, 26.25, 28.4, 28.57,30.77, 32.08, 32.78, 34.10, 35.50, 36.73, 38.48, 39.59, 43.38, 45.3 as depicted in Fig.7,

iii) the IR spectrum is substantially in accordance with Fig. 8,

iv)a DSC thermogram substantially in accordance with Fig. 9,

v) DSC thermogram shown in Fig.7 points melting point: 216.74 and 224.70 for polymorph P of imatinib mesylate.

In another aspect of the present invention, polymorphs Y, B, M and P have superior good flow properties and stability due to their low hygroscopicity.

In the present invention, samples of obtained crystalline forms are stored for one month in stability chambers under following conditions of 40°C and humidity of 75%, for 3 months to support non-hygroscopic nature of novel forms of imatinib mesylate.

Non-hygroscopicity test results are listed below in table 1.

**Table 1**

| **Sample** | **Initial Water Content** (%) | **Water Content after 3 months** (%) |
|---|---|---|
| Polymorph Y sample | 0.40 | 0.50 |
| Polymorph B sample | 0.37 | 0.48 |
| Polymorph M sample | 0.38 | 0.48 |
| Polymorph Psample | 0.42 | 0.50 |
| Polymorph β reference sample | 0.34 | 0.60 |
| Condition: 40°C at humidity of 75%, for 3 months | | |

In another aspect of the present invention, polymorphs Y, B, M, P of Imatinib and a reference standard material equivalent to the one referred in the previous section are treated with water at 25°C.

According to the present invention, as seen in table 2, inventors have discovered that novel crystalline forms of imatinib mesylate are highly soluble than reference β polymorph sample of imatinib mesylate.

The solubility results are summarized below in table 2.

**Table 2**

| **SAMPLE** | **FORM** | **SOLUBILITY** (mg/ml) |
|---|---|---|
| 1 | Polymorph Y sample | ≥ 2000 |
| 2 | Polymorph B sample | ≥ 2000 |
| 3 | Polymorph M sample | ≥ 2000 |
| 4 | Polymorph P sample | ≥ 2000 |
| 5 | Polymorph β reference sample | ≥ 1200 |

Yet another objective of the present invention is to provide novel pharmaceutical compositions containing the novel polymorphic forms of imatinib mesylate or their mixtures.

The compositions containing the novel crystalline polymorphic forms of imatinib mesylate may include fillers, binders, disintegrants, glidants, lubricants, flavourings.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches (including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested. Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Flavourings are, but not limited to, cinnamon oil, essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehydeacacia, anise oil, benzaldehyde, caraway oil, cardamom (oil, tincture, spirit),cherry syrup, citric acid syrup, citric acid, clove oil, cocoa, cocoa syrup, coriander oil, dextrose, ethyl acetate, ethyl vanillin, fennel oil, ginger, glucose, glycerin, glycerrhiza, honey, lavender oil, lemon oil, mannitol, nutmeg oil, methyl salicylate, orange oil, orange flower water, peppermint (oil, spirit, water), raspberry, rose (oil, water), rosemary oil, saccharin sodium, Sarsaparilla syrup, spearmint oil, thyme oil, tolu balsam, vanilla ,vanilla tincture, tolu balsam syrup, wild cherry syrup and mixtures thereof and the like.

Selection of excipients and amounts to use can be determined by the scientist. One or more these additives can be chosen and used by the artisan having regard to the particular desired properties of the solid dosage form.

Suitable pharmaceutical compositions include, but are not limited to, capsules, tablets, granules, powders and unit dose pockets.

The following example is given for the purpose of illustration of the present invention and should not limit the scope of the invention.

**Table 3**

| **PERCENTAGES BASED ON BY WEIGHT OF TOTAL PHARMACEUTICAL COMPOSITION** | | |
|---|---|---|
| | **mg/tablet** | %**(w/w)** |
| **Ingredients** | | |
| Imatinib (Mesylate Salt) | 400 (478) | 25.97 |
| Microcrystalline Cellulose(PH 101) | 290.0 | 18.83 |
| Hydroxypropyl methylcellulose (HPMC) | 70 | 4.54 |
| Microcrystalline Cellulose(PH 102) | 385 | 25.00 |
| Crospovidone | 280 | 18.2 |
| Colloidal Silicon Dioxide | 50 | 3.22 |
| Magnesium Stearate | 30 | 2 |
| Water** | q.s. | |
| **Total Core Tablet Weight** | **1.505.0** | **97.72** |
| Polyvinyl alcohol | 14.00 | 0.90 |
| Polyethylene glycol (Macrogol)/PEG 3350 | 7.07 | 0.45 |
| Yellow iron oxide | 6.65 | 0.43 |
| Talc | 5.18 | 0.33 |
| Titanium Dioxide | 1.75 | 0.11 |
| Red Iron Oxide | 0.35 | 0.02 |
| Water | q.s. | |
| **Total Film Coating Weight** | **35.00** | **2.27** |
| **Total Tablet Weight** | **1540.00** | **100.00** |

The tablets are prepared by wet granulation of Imatinib mesylate, microcrystalline cellulose, hydroxypropyl methyl cellulose, crospovidone, colloidal silicon dioxide with water. Then resultant tablets are compressed and coated with an aqueous dispersion of the coating mixture.

During manufacturing of formulation prepared with new polymorphs (Y, B, M, P) have shown good hygroscopic properties under controlled moisture environment and has considerably higher solubility in an aqueous medium.

### Example 1: PREPARATION OF CRYSTALLINE M FORM IMATINIB MESYLATE

Imatinib base (2.5 g) is suspended in acetone of 35 ml. The mixture is heated to reflux temperature under a nitrogen atmosphere for 20 minutes. To this mixture, methane sulfonic acid 0.326 ml in acetone (5 ml) is added slowly over 1 hour under a nitrogen atmosphere. The mixture is refluxed for 30 minutes and filtered at 50-55°C.

The crystals are dried under vacuum for about 4 hours at 55-60°C, to obtain 2.2 g of crystalline mixture form imatinib mesylate.

### Example 2: PREPARATION OF CRYSTALLINE Y-FORM IMATINIB MESYLATE

Imatinib base (2.0 g) is suspended in propanol of 30 ml. The mixture is cooled to 0-5 °C for 30 minutes. To this mixture, 0.263 ml methanesulfonic acid is added slowly over 20 minutes. At the end of the addition of methanesulfonic acid, the solution becomes clear and immediately crystals start to precipitate. The mixture is stirred further 30 minutes at this temperature and filtered.

The crystals are dried under vacuum for about 4 hours at 55-60°C, to obtain 1.9 g of crystalline Y form imatinib mesylate.

### Example 3: PREPARATION OF CRYSTALLINE B-FORM IMATINIB MESYLATE

Imatinib base (2.0 g) is suspended in THF of 50 ml at room temperature. To this mixture, methanesulfonic acid 0.263 ml in THF (50 ml) is added slowly over 1 hour under a nitrogen atmosphere at room temperature. The mixture is heated to 55-60°C under a nitrogen atmosphere for 30 minutes. The obtained suspension is cooled to room temperature and the resulting wet crystals of imatinib mesylate B form is filtered and dried under vacuum for about 4 hours at 55-60°C, to obtain 1.9g of B form imatinib mesylate.

### Example 4: PREPARATION OF CRYSTALLINE P FORM IMATINIB MESYLATE

Imatinib base (3.5 g) is suspended in THF of 90 ml. The mixture is heated to 55-60°C under a nitrogen atmosphere for 20 minutes. The mixture becomes clear. To this mixture, α-form Imatinib mesylate 50 mg is added. A solution of methanesulfonic acid (0.460 ml) in THF (50 mL) is added slowly over 2 hours under a nitrogen atmosphere. The mass is stirred for another 30 minutes at 55-60°C. The mixture is cooled to 30°C and filtered at this temperature.

The crystals are dried under vacuum for about 4 hours at 55-60°C, to obtain 2.2 g of crystalline mixture form imatinib mesylate.

## Claims

1. A new crystalline imatinib mesylate crystalline M form, is **characterized by** a x-ray powder diffraction spectrum having peaks at about expressed as 2θ at about 5.97,9.64,10.42,10.94,11.59,12.93,13,916,14.63,15.567,17.39,18.051,18.82,1 9.90, 20.51, 21.04, 21.8, 21.97, 22.63, 23.66, 24.2, 26.23, 26.8, 28.3, 29.10, 29.61, 30.1, 30.76, 31.79, 32.76, 34.02, 35.52, 36.80, 40.5, 42.2,43.5 degrees.

2. According to claim 1, imatinib mesylate crystalline M form is obtained with the reaction of equimolar amount of imatinib base and methanesulfonic acid, in a ketone.

3. A method of producing crystalline M form of imatinib mesylate as defined in claim 1, said method is consisting of the following steps: (a) Suspending or dissolving imatinib base in a ketone.
(b) heating the mixture for reflux temperature,(c) adding methanesulfonic acid, at a controlled rate, diluted with solvent, to the reaction mixture,(d) cooling the mixture to ambient temperature and isolating the mixture crystal form;(e) isolating the crystalline product from the reaction mixture(f) drying the isolated product under vacuum at temperature from 50 to 65°C.

4. A new crystalline imatinib mesylate crystalline Y form, is **characterized by** an x-ray powder diffraction spectrum having peaks expressed as 2θ at about 6.017, 9.67,10.40, 10.95,11.6, 12.91, 13.950, 14.640,17.09,17.4,18.09,19.97, 20.54, 21.03, 22.01, 23.66, 24.2, 26.23, 29.11, 29.61, 30.77, 31.74, 32.75, 34.05, 35.55, 36.81, 38.42, 39.02, 40.48, 43.46, 45.28, 48.9.degrees.

5. According to claim 4, imatinib mesylate crystalline Y form is obtained with the reaction of equimolar amount of imatinib base and methanesulfonic acid, in an alcohol.

6. A method of producing crystalline Y form of imatinib mesylate as defined in claim 4, said method is consisting of the following steps:(a) suspending imatinib base in a alcohol and stirring the solution at temperature from -10 to 10°C, (b) adding methanesulfonic acid, at a controlled rate, diluted with solvent, to the reaction mixture, (c) stirring the reaction mixture at temperature from -10°C to 10°C, (d) isolating the crystalline product from the reaction mixture, (f) drying the isolated product under vacuum at temperature from 50 to 65°C.

7. A new crystalline imatinib mesylate of crystalline B form, is **characterized by** an x-ray powder diffraction spectrum having peaks expressed as 2θ at about 4.7, 9.67, 10.44, 10.95, 13.0, 13.92, 14.65, 17.39, 18.10, 19.96, 20.55, 21.04, 22.03, 22.6267, 23.68, 25.06, 26.27, 26.78, 28.3, 29.64, 30.75, 31.75, 32.78, 34.04, 35.56, 36.83 degrees.

8. According to claim 7, imatinib mesylate of crystalline B form is obtained with the reaction of equimolar amount of imatinib base and methanesulfonic acid in an ether.

9. A method of producing crystalline B form of imatinib mesylate as defined in claim 7, said method is consisting of the following steps: (a) suspending or dissolving imatinib base in a ether and stirring the solution at room temperature,
(b) adding methanesulfonic acid, at a controlled rate, diluted with solvent, to the reaction mixture, (c) stirring the reaction mixture at room temperature, (d) heating the above mixture for a sufficient amount of time to induce the formation of new crystal form;(e)cooling the mixture to ambient temperature and isolating the new crystal form; (f) isolating the crystalline product from the reaction mixture, (g) drying the isolated product under vacuum at temperature from 50 to 65°C.

10. A new crystalline imatinib mesylate of P , is **characterized by** an x-ray powder diffraction spectrum having peaks expressed as 2θ at about about 4.92, 9.69, 10.45, 12.00, 12.95, 13.90, 14.70, 17.36, 18.06, 18.52, 19.08,19.93, 20.51, 20.97, 21.66, 22.0,24.85, 26.25, 28.4, 28.57,30.77, 32.08, 32.78, 34.10, 35.50, 36.73, 38.48, 39.59, 43.38, 45.3 degrees.

11. According to claim 10, imatinib mesylate of crystalline P form is obtained with the reaction of equimolar amount of imatinib base and methanesulfonic acid, a cyclic ether.

12. A method of producing a crystalline P form as defined in claim 10, said method is consisting of the following steps: (a) Suspending or dissolving imatinib base in a ether and stirring the solution at room temperature, (b) Stirring the reaction mixture at room temperature,(c) Heating the above mixture for reflux temperature (d) Adding alpha crystals of imatinib mesylate.(e) Adding methanesulfonic acid, at a controlled rate, diluted with solvent, to the reaction mixture,(f) Cooling the mixture to ambient temperature and isolating the mixture crystal form; (g) Isolating the crystalline product from the reaction mixture, (h) Drying the isolated product under vacuum at temperature from 50 to 65°C.
